# EUROPEAN PATENT SPECIFICATION

(11) **EP 4 288 030 B1**
(45) Date of publication and mention of the grant of the patent: **09.07.2025**
(21) Application number: 22704513.5
(22) Date of filing: 31.01.2022
(51) Int. Cl.: A61K 9/00, A61K 9/10, A61K 47/36, A61K 47/38

(54) **AN AQUEOUS GEL COMPOSITION COMPRISING AN ETHYLCELLULOSE**
WÄSSRIGE GELZUSAMMENSETZUNG MIT ETHYLCELLULOSE
COMPOSITION DE GEL AQUEUX COMPRENANT UNE ÉTHYLCELLULOSE

(30) Priority: 02.02.2021 IN 202111004454
(43) Date of publication of application: 13.12.2023
(73) Proprietor: International N&H USA, Inc., Wilmington, DE 19803 (US); Danisco Nutrition and Biosciences India Pvt. Ltd., Greater Kailash-1 New Delhi (IN)
(72) Inventor: TORNE, Sangmesh, New Delhi (IN); BHAGAT, Ajit, New Delhi (IN); GUNJIKAR, Tejas, New Delhi (IN)
(74) Representative: f & e patent
(86) International application number: PCT/EP2022/052182
(87) International publication number: WO 2022/167359

(56) References cited:
- WO-A1-2011/032728
- WO-A1-2017/120492
- US-A1- 2014 010 884

## Description

### FIELD

The present invention relates to novel aqueous gel compositions comprising a physiologically active ingredient in an aqueous dispersion of ethylcellulose comprising a hydrophilic gelling agent.

### INTRODUCTION

Aqueous dispersions of ethylcellulose have been developed and used for many years for coatings on solid dosage forms providing sustained release of active ingredients from the dosage form in the intestines.

In 1993 Oshlack, Benjamin et al. (US5472712) disclosed the use of Aquacoat^{®} ECD30 for controlled release of hydromorphone from an oral dosage form. In 2004, Beyerink R A (US10383941), reported the use of ethylcellulose for controlled release of an active ingredient from an oral dosage form by encapsulation using spray drying technique.

Duprat A. (US9375477) disclose a topical gel preparation using carrageenan as a gelling agent without including a hydrophobic polymer like ethylcellulose for controlled release action.

Arnaud (US590863) discloses the use of ethylcellulose as gelling agent in presence of organic solvents and also mention that the ethyl cellulose is a hydrophobic polymer with limited solubility in water. Goyal Sandhya et al. (US8158139) disclose ethylcellulose in topical gel formulations including organic solvent. Chellampillai et al., Ther. Deliv. 2014 Jul;5(7):781-94; studied the use of ethylcellulose in development of microsponges for topical carriers for the controlled release and cutaneous drug deposition of eberconazole nitrate (EB) in antifungal therapy using an organic solvent. Lilia Bruno et al., Int. J. Biol. Macro-mol. 50(2), 1 March 2012, pp. 385-392; studied the effect of hydration on the structure of a nonaqueous ethylcellulose gel for topical application. Sanjukta Duarah et al., 2017 Oct;7(5):750-760, Drug Deliv. Transl. Res. used ethylcellulose to develop nanoparticles in a gel delivery system using an organic solvent.

US2014/010884 relates to a topically applicable, chemically, physically and rheologically stable dermatological composition suited for treating dermatological conditions and afflictions linked to disorders of cell differentiation and/or proliferation and/or keratinization, comprising thus effective amounts of at least one retinoid, dispersed benzoyl peroxide and at least one carrageenan gelling agent, formulated into a topically applicable, physiologically acceptable medium therefore.

WO2017/120492 relates to a gel composition comprising: (a) at least one active ingredient or a salt thereof; (b) at least one oleogel comprising at least one oily agent and at least one lipid soluble cellulose polymer, wherein the at least one oily agent comprises from about 5% to about 40% by weight of the total weight of the composition and the at least one cellulose polymer comprises from about 1 % to about 10% by weight of the total weight of the composition; and (c) at least one aqueous gel.

WO2011/032728 relates to a hair styling gel comprising, per 100 parts by weight of said gel: - from 10 to 20 parts by weight of a plasticizer, - from 2 up to 3 parts by weight of a carrageenan mixture, - from 0.1 to 1 .0 parts by weight of a preservative, and water forming the balance, characterised in that said carrageenan mixture comprises 60% to 95% by weight of iota-carrageenans and 5% to 25% by weight of lambda-carrageenans, and wherein said hair styling gel is free from synthetic polymers.

Thus, ethylcellulose is known for its insolubility in aqueous media and is used in topical gel formulations with an organic solvent as a base. While ethylcellulose solutions in organic solvents impart higher viscosity, ethylcellulose has a very low solubility in water and cannot function on its own as gelling polymer controlling the release rate of an active ingredient in an aqueous gel formulation for topical application.

The objective of the present invention is to provide an aqueous gel formulation from which an active ingredient is released at a controlled rate on topical application.

### SUMMARY OF THE INVENTION

It has surprisingly been found that when a hydrophilic gelling agent is added to an aqueous dispersion of ethylcellulose, it is possible to obtain an aqueous gel in which an active ingredient may be incorporated and from which it is released at a controlled rate.

Accordingly, the present invention relates to an aqueous gel composition for topical application comprising
(a) a physiologically active ingredient,
(b) an aqueous dispersion of ethylcellulose, and
(c) a carrageenan,
wherein the concentration of ethylcellulose is in the range of 5-50% by weight of the composition.

In another aspect, the invention relates to a method of preparing the above composition, the method comprising
(i) heating an aqueous dispersion of ethylcellulose to a temperature of 70-90°C,
(ii) adding a carrageenan to the dispersion of step (i) with continuous mixing until a gel is formed,
(iii) cooling the mixture of step (ii) to a temperature of 25-50°C and adding the physiologically active ingredient with mixing, and
(iv) cooling the mixture of step (iii) to a temperature of 20-25°C and optionally adding one or more auxiliary ingredients to the gel.

### BRIEF DESCRIPTION OF THE DRAWINGS

Fig. 1 A-E are photographs showing the spreadability of gel formulations prepared in Example 1.
Fig. 2 is a graph showing the storage modulus (in Pa) of gel formulations prepared in Example 1 and with the results described in Example 3.

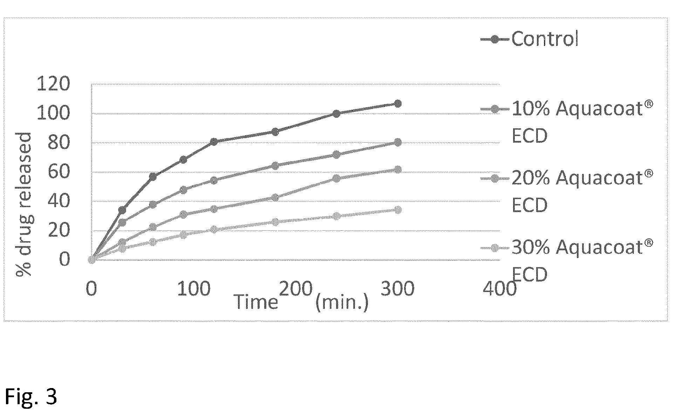

Fig. 3 show the cumulative release of Diclofenac Sodium from Topical Gel prepared according to the method described in Example 1 at 7.4 pH Phosphate Buffer, Temperature 32⁰C with the results also described in Table 4 of Example 4.

### DETAILED DESCRIPTION OF THE INVENTION

### Definitions

In the present context, the term "topical application" is intended to indicate spreading the gel composition on a body surface such as skin, mucosal epithelium or nails.

The term "gel" is intended to indicate a semisolid dosage form containing one or more gelling agents that provide increased viscosity to a solution or dispersion of solid particles.

The term "dispersion" is intended to indicate a composition of dispersed particles, e.g. particles of ethylcellulose and insoluble active ingredients distributed homogenously throughout a continuous liquid medium such as water.

The term "controlled release" is intended to indicate that a finite quantity of an active ingredient is released at a specific rate from the gel composition over an extended period of time. The term is synonymous with the term "sustained release".

### Embodiments

Ethylcellulose is a cellulosic polymer / polysaccharide composed of anhydroglucose units joined by 1 - 4 linkages. Each anhydroglucose unit contains hydroxyl groups at the 2, 3 and 6 positions. Partial or complete substitution of these hydroxyl groups by ethoxyl groups yields ethylcellulose. Ethylcellulose typically comprises 44-51% ethoxyl groups according to the USP monograph. The aqueous dispersion of ethyl cellulose is prepared by polymeric emulsion process. The ethyl cellulose in powder form is dissolved in organic solvent emulsified with water in presence of surfactant and one or more process aids, and the organic solvent is subsequently removed resulting in the formation of ehtylcellulose particles suspended in water as disclosed in US 4,177,177. The concentration of ethylcellulose in the present composition is suitably in the range of 5-50% by weight of the composition.

Examples of suitable ethylcelluloses are marketed by DuPont under the trade name ETHOCEL^{™}. A commercially available ethylcellulose aqueous dispersion is available from DuPont under the trade name AQUACOAT^{®} ECD30 and from Colorcon under the trade name Surelease^{®}. AQUACOAT^{®} ECD is an aqueous dispersion containing 24.5%-29.5% by weight ethylcellulose, 0.9-1.7% by weight sodium lauryl sulfate and 1.7-3.3% by weight cetyl alcohol. Cetyl alcohol is an emulsifier which acts to stabilize the dispersion of ethylcellulose in water.

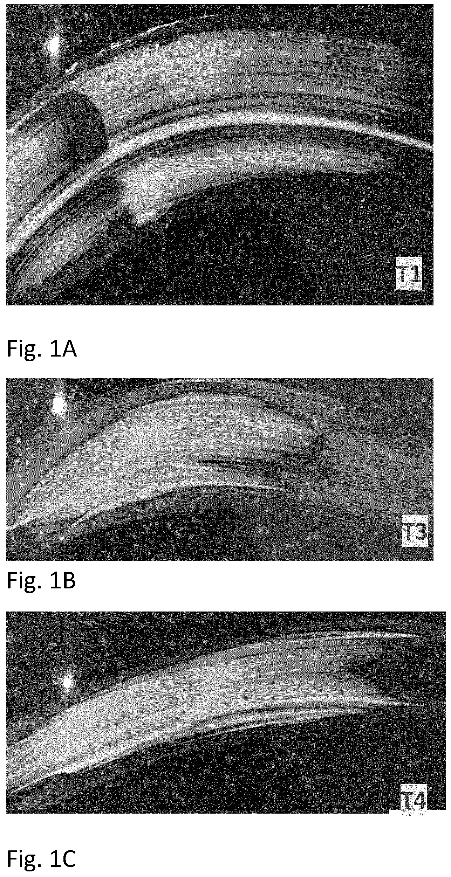

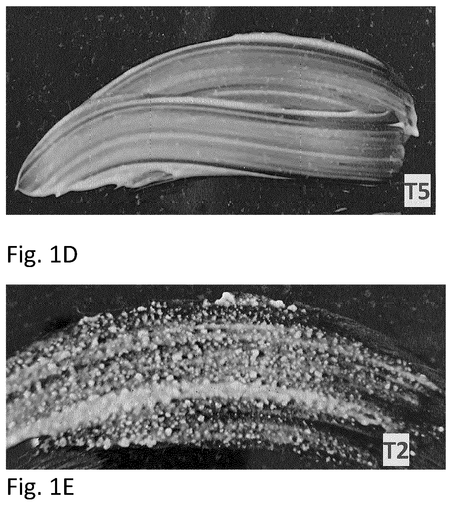

Carrageenans are hydrophilic polysaccharides found in red seaweeds. Carrageenans contain repeating galactose units joined by alternating α1→3 and β1→4 glycosidic linkages. The carrageenan's of interest to this invention are kappa-, kappa-2-, iota- and lambda- carrageenan. Iota-carrageenan is found in the seaweed *Eucheuma denticulatum,* while kappa-carrageenan may be extracted from the seaweed *Eucheuma cotonii* and kappa-2-carrageenan, which is a copolymer of kappa- and iota-carrageenan repeating units covalently bound in the copolymer backbone, may be obtained from *Gigartinaceae* species. Carrageenan acts as a supplementary gelling agent in the gel composition with a mechanism of aggregation of helical dimers. It absorbs water to swell and form a polymeric network and dispersed ethyl cellulose is spread across the network. For the present purpose, iota-carrageenan (such as Gelcarin^{®} PH379 available from DuPont) has been found to be particularly useful as it imparts good spreadability to the gel composition (cf. Fig. 1 A - D appended hereto). The concentration of carrageenan in the gel composition is preferably in the range of 0.1-5% by weight of the composition.

The viscosity of the aqueous gel composition of the invention is typically in the range of 100 - 6000 mPa.s at a shear rate of 0.01 s⁻¹ as determined using a rheometer (model Discovery HR-3, available from TA Instruments) as follows: the 40mm flat plate was used to apply continuous shear sweep at a rate of 0.01 s⁻¹ to 100s⁻¹. The viscosity was measured at 0.01 s⁻¹ shear rate.

The gel of the invention generally has a yield stress in the range of 10 - 2000 Pa as determined using a rheometer (model Discovery HR-3, available from TA Instruments). The yield stress is a lowest shear stress above which a test material behaves like a fluid. Or alternatively a minimum force required to break the structure of a gel at rest and thus make it flow and be applied easily on skin. Yield stress is measured as the oscillation stress at which a sample loses storage modulus significantly and is reported in Pa.

The physiologically active ingredient may be any pharmacologically or cosmeceutically active ingredient suitable for application on a body surface such as the skin and which may beneficially be released to the site of application in a controlled manner. Active ingredients which may be included in the present gel composition may be selected from the group consisting of antibiotics (e.g. clindamycin, erythromycin), antimycotic agents (e.g. ketoconazole, fluconazole, clotrimazole), antiviral agents (e.g. acyclovir, famciclovir, ), non-steroidal anti-inflammatory agents (e.g. diclofenac sodium, ibuprofen, ketoprofen ), local anesthetics (e.g. lidocaine, bupivacaine), corticosteroids (e.g. hydrocortisone, betamethasone, clobeta-sol), anti-allergic agents, anticancer agents, hair growth stimulants, immunosuppressants (e.g. tacrolimus, ciclosporin), antidiabetic agents, antidepressants , vitamins (e.g. vitamin A, vitamin D, vitamin E) , sunscreen agents, alopecia treatments, anti-acne agents (e.g. clindamycin, benzoyl peroxide, retinoids), antipsoriatic agents and wound healing agents.

The gel composition of the invention may further comprise one or more auxiliary ingredients selected from the group consisting of pH modifiers, preservatives, surfactants, emollients, penetration enhancers, and optionally fragrances.

Examples of pH modifiers include bases such as hydroxides, carbonates, citrates and phosphates, as well as salts thereof such as sodium citrate. The amount of the pH modifier may vary depending on the desired pH of the gel composition. The pH of the gel composition may be in the range of 3.0-8.0, but is preferably close to a neutral pH, preferably in the range of 5.5 - 7.0.

Examples of preservatives include benzalconium chloride, benzethonium chloride, chlorhexidine, sodium benzoate, sorbic acid, potassium sorbate, BHA or BHT

Examples of surfactants include anionic surfactants, including alkyl sulfates such as sodium lauryl sulfate or sodium dodecyl sulfate, non-ionic surfactants such as polysorbates, fatty acid ester of propylene glycol, fatty acid esters of glycerol or fatty acid esters of polyethylene glycol. The surfactant may be included in a concentration of 0.2% - 2% by weight of the gel composition, preferably 0.5% - 1.7% by weight of the gel composition. A preferred surfactant for inclusion in the present gel composition is sodium lauryl sulfate. The aqueous dispersion of ethylcellulose may further include an emulsion stabilizer such as a fatty alcohol, e.g. cetyl alcohol.

Examples of emollients include cetyl alcohol, cetearyl alcohol, stearyl alcohol, isopropyl myristate, isopropyl palmitate, lanolin, liquid paraffin, polyethylene glycols, silicone oils or stearic acid.

Examples of penetration enhancers include propylene glycol, Myrj 52, Cineol, dimethyl sulfoxide, dimethyl isosorbide, isopropyl myristate, menthol, peppermint oil.

It has surprisingly been found that the aqueous gel composition of the invention not only exhibits advantageous gelation and spreadability properties due to the presence of carrageenan therein, but also provides for controlled release of the active ingredient due to the ethylcellulose present in the gel. As ethylcellulose is a hydrophobic polymer it retards the migration of active ingredients from the gel into the skin. The present inventors have observed that the active ingredient is released from the gel in a continuous manner over an extended period of time such as a period of at least 4 hours, preferably at least 5 hours. The release of the active ingredient has been found to be dependent on the concentration of ethylcellulose in the gel, cf. Example 4 below.

The invention is further described in the following examples.

### EXAMPLES

### Example 1

### Aqueous gel compositions containing 1% by weight diclofenac sodium

Aqueous gel compositions were prepared with the composition apparent from Table 1.

**Table 1**

| **Ingredient** | **T1 (% w/w)** | **T2 (% w/w)** | **T3 (% w/w)** | **T4 (% w/w)** | **T5 (%w/w)** |
|---|---|---|---|---|---|
| AQUACOAT^{®} ECD 30¹ | 99 | 99 | 89 | 78.8 | 88.9 |
| Gelcarin^{®} PH379² | 1 | 0 | 1 | 1.2 | 1.1 |
| Gelcarin^{®} PH812³ | 0 | 1 | 0 | 0 | 0 |
| Water | 0 | 0 | 10 | 20 | 10 |
| Total | 100 | 100 | 100 | 100 | 100 |

| | | | | | |
|---|---|---|---|---|---|
| 1. Aqueous dispersion containing 30% w/w of ethylcellulose 2. Iota-carrageenan 3. Kappa-carrageenan | | | | | |

The aqueous dispersion of ethylcellulose and optionally additional water was heated to 70°C, and carrageenan was added with continuous mixing at 150 to 200 rpm until the gel was formed. The gel was cooled to 40°C and diclofenac sodium was added with continuous mixing at 150 to 200 rpm to a concentration of 1% w/w. The gel was allowed to cool to 20°C. The resulting gels were opaque and white in colour.

The pH of the finished gels was measured to be: T1, pH 6.33; T2, pH 6.04; T3, pH 6.38; T4, 6.30; T5, pH 6.24, i.e. close to a neutral pH.

### Example 2

### Spreadability of the gel compositions of Example 1

The spreadability of the gel compositions of Example 1 was determined by visual observation of plate method. The prepared gels were applied on black tiles using an index finger and spread across the surface, very similar to application on skin. The formed film was observed visually to understand the uniformity in gel.

The results are shown in Fig. 1 A-E from which it appears that the gel compositions T1 and T3-T5 that include iota-carrageenan have a better spreadability than the gel composition T2 that includes kappa-carrageenan.

### Example 3

### Rheology of gel compositions of Example 1

Samples from compositions of example 1 were used to apply oscillation strain of 0.001 to 1000% using a rheometer model Discovery HR-3 available from TA instruments, and storage modulus was measured against each strain. The storage modulus was measured at 0% strain which is 40 to 80 seconds after the start of the test. The storage modulus is a measure of the resilience of the gel structure.

The results are shown in Table 2.

**Table 2**

| **Storage modulus (Pa) at 60 seconds** | |
|---|---|
| T1 | 674 |
| T2 | 843 |
| T3 | 181 |
| T4 | 120 |
| T5 | 431 |

It appears from Table 2 that the gel composition T4 has the lowest shear thinning of the gel compositions prepared in Example 1, making it easy to apply on a skin surface.

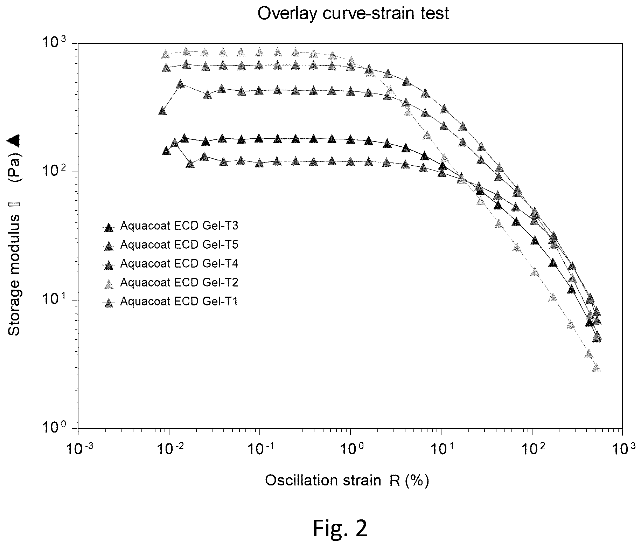

The results of the overlay curve-strain test are shown in Fig. 2, from which it appears that the gel typical achieves a storage modulus from 100 to 1000 Pa. The straight line of storage modulus showed the initial resilience of gel, and the curve showing a decline in the storage modulus confirmed the disruption of gel structure. The decline of the storage modulus and equivalent applied strain confirmed the minimum required strain for each formulation to break its gel structure which happens on application on skin. The formulation (T4) was determined to have a lower storage modulus which makes the gel easy to apply while at the same time maintaining its integrity as a gel.

### Example 4

### Aqueous gel compositions containing 1% w/w diclofenac sodium for release studies

Aqueous gel compositions were prepared with the composition apparent from Table 3.

**Table 3**

| **Ingredient** | **T6 (% w/w)** | **T7 (% w/w)** | **T8 (% w/w)** | **T9 (% w/w)** |
|---|---|---|---|---|
| AQUACOAT^{®} ECD 30 | 0 | 78 | 88 | 98 |
| Gelcarin^{®} PH379 | 1 | 1 | 1 | 1 |
| Diclofenac sodium | 1 | 1 | 1 | 1 |
| Water | 0 | 20 | 10 | 0 |
| Total | 100 | 100 | 100 | 100 |

The gel compositions were prepared according to the method described in Example 1.

Static jacketed Franz-type diffusion cells with an available diffusion area of 700 cm² and receptor volumes ranging from 10-12 ml were used in substantially the manner described by T.J. Franz, "The finite dose technique as a valid in vitro model for the study of percutaneous absorption in man" in Current Problems in Dermatology, 1978, J.W.H. Mall (Ed.), Karger, Basel, pp. 58-68. An oval magnetic bar (length 10 mm, width 6 mm) was placed in the receptor compartment of each cell. After mounting the membrane, receptor medium (0.04 M isotonic phosphate buffer, pH 7.4) was filled into the receptor chamber. The temperature of the dissolution media in the receptor chamber was maintained at 32°C by continuously circulating hot water. The oval magnetic stirrer placed at the bottom of the receptor chamber was set to stir at 500rpm.

Each of the test compositions T6 - T9 was applied on the membrane with a spatula at 0 hours in an intended dose of 300mg equivalent to 3mg diclofenac sodium / 700cm².

Samples (1 ml) of the receptor fluid were collected at 0, 30, 60, 90, 120, 180, 240 and 300 minutes and analyzed for content of diclofenac sodium by ultraviolet spectroscopy method at 270nm. The absorbance of each sample at the specified wavelength was used to calculate drug release against the standard curve of absorbance. The percent drug release from each sample at different time interval is summarized in in Table 4.

**Table 4: Release of diclofenac sodium over time**

| **Time (min.)** | **T6 Control** | **T7 10% EC** | **T8 20% EC** | **T9 30% EC** |
|---|---|---|---|---|
| 0 | 0 | 0 | 0 | 0 |
| 30 | 34.05 | 25.62 | 12.01 | 7.79 |
| 60 | 56.66 | 37.64 | 22.28 | 12.23 |
| 90 | 68.37 | 47.77 | 30.89 | 17.09 |
| 120 | 80.59 | 54.18 | 34.67 | 20.69 |
| 180 | 87.42 | 64.16 | 42.47 | 25.78 |
| 240 | 99.75 | 71.74 | 55.54 | 29.72 |
| 300 | 106.69 | 80.24 | 61.68 | 34.2 |

It appears from Table 4 that diclofenac sodium is released in a controlled manner from the gel compositions and that the concentration of ethylcellulose has a pronounced impact on the release rate.

## Claims

1. An aqueous gel composition for topical application comprising
(a) a physiologically active ingredient,
(b) an aqueous dispersion of ethylcellulose, and
(c) a carrageenan,
wherein the concentration of ethylcellulose is in the range of 5-50% by weight of the composition.

2. The composition of claims 1, wherein the concentration of carrageenan is in the range of 0.1-5% by weight of the composition.

3. The composition of any one of claims 1-2, wherein the carrageenan is selected from the group consisting of iota-carrageenan, kappa-carrageenan, kappa-2-carrageenan and lambda-carrageenan.

4. The composition of claim 3, wherein the carrageenan is iota-carrageenan.

5. The composition of any one of claims 1-4, wherein the physiologically active ingredient is selected from the group consisting of antibiotics, antimycotic agents, antiviral agents, non-steroidal anti-inflammatory agents, local anesthetics, corticosteroids, retinoids, anti-allergic, anticancer agents, hair growth stimulants, immunosuppressants, antidiabetic agents, antidepressants, vitamins, sunscreen agents, alopecia treatment, anti-acne agents, anti-psoriatic agents and wound healing agents.

6. The composition of any one of claims 1-5, wherein the gel has a viscosity of 100 to 6000 mPa.s at shear rate of 0.01 s⁻¹ as determined using a rheometer (model Discovery HR-3, available from TA Instruments).

7. The composition of any one of claims 1-6, wherein the gel has a yield stress of 10 to 200 Pa determined as storage modulus using a rheometer (model Discovery HR-3, available from TA Instruments).

8. The composition of any one of claims 1-7 which has a pH in the range of 3-8.

9. The composition of any one of claims 1-8 further comprising an auxiliary ingredient selected from the group consisting of pH modifiers, preservatives, surfactants, emollients, penetration enhancers, optionally fragrances.

10. The composition of any one of claims 1-9 providing controlled release of the physiologically active ingredient on topical application thereof.

11. A method of preparing the composition of any one of claims 1-10, the method comprising
(i) heating an aqueous dispersion of ethylcellulose to a temperature of 50-80°C,
(ii) adding a carrageenan to the dispersion of step (i) with continuous mixing until a gel is formed,
(iii) cooling the mixture of step (ii) to a temperature of 25-50°C and adding the physiologically active ingredient with mixing, and
(iv) cooling the mixture of step (iii) to a temperature of 20-25°C and optionally adding one or more auxiliary ingredients to the gel.

## Patentansprüche

1. Wässrige Gelzusammensetzung zur topischen Anwendung, umfassend
(a) einen physiologischen Wirkstoff,
(b) eine wässrige Dispersion von Ethylcellulose und
(c) ein Carrageenan,
wobei die Konzentration an Ethylcellulose im Bereich von 5-50 Gew.-% der Zusammensetzung liegt.

2. Zusammensetzung nach Anspruch 1, wobei die Konzentration an Carrageenan im Bereich von 0,1-5 Gew.-% der Zusammensetzung liegt.

3. Zusammensetzung nach einem der Ansprüche 1-2, wobei das Carrageenan aus der Gruppe bestehend aus iota-Carrageenan, kappa-Carrageenan, kappa-2-Carrageenan und lambda-Carrageenan ausgewählt ist.

4. Zusammensetzung nach Anspruch 3, wobei es sich bei dem Carrageenan um iota-Carrageenan handelt.

5. Zusammensetzung nach einem der Ansprüche 1-4, wobei der physiologische Wirkstoff aus der Gruppe bestehend aus Antibiotika, Antimykotika, antiviralen Mitteln, nichtsteroidalen Antiphlogistika, Lokalanästhetika, Kortikosteroiden, Retinoiden, Antiallergika, Antikrebsmitteln, Mitteln, die das Haarwachstum stimulieren, Immunsuppressiva, Antidiabetika, Antidepressiva, Vitaminen, Sonnenschutzmitteln, Alopeziebehandlung, Antiaknemitteln, Antipsoriasismitteln und Wundheilungsmitteln ausgewählt ist.

6. Zusammensetzung nach einem der Ansprüche 1-5, wobei das Gel eine Viskosität von 100 bis 6000 MPa.s bei Scherrate von 0,01 s⁻¹, wie unter Verwendung eines Rheometers (Modell Discovery HR-3, erhältlich von TA Instruments) bestimmt, aufweist.

7. Zusammensetzung nach einem der Ansprüche 1-6, wobei das Gel eine Fließspannung von 10 bis 200 Pa, bestimmt als Speichermodul unter Verwendung eines Rheometers (Modell Discovery HR-3, erhältlich von TA Instruments), aufweist.

8. Zusammensetzung nach einem der Ansprüche 1-7, die einen pH-Wert im Bereich von 3-8 aufweist.

9. Zusammensetzung nach einem der Ansprüche 1-8, ferner umfassend einen Hilfsbestandteil, der aus der Gruppe bestehend aus Mitteln zur Modifizierung des pH-Werts, Konservierungsmitteln, Tensiden, Emollientien, Penetrationsverstärkern, gegebenenfalls Duftstoffen, ausgewählt ist.

10. Zusammensetzung nach einem der Ansprüche 1-9, die bei topischer Anwendung eine kontrollierte Freisetzung des physiologischen Wirkstoffs bereitstellt.

11. Verfahren zur Herstellung der Zusammensetzung nach einem der Ansprüche 1-10, wobei das Verfahren Folgendes umfasst:
(i) Erhitzen einer wässrigen Dispersion von Ethylcellulose auf eine Temperatur von 50-80 °C,
(ii) Zugeben eines Carrageenans zu der Dispersion von Schritt (i) unter kontinuierlichem Mischen, bis sich ein Gel bildet,
(iii) Abkühlen der Mischung aus Schritt (ii) auf eine Temperatur von 25-50 °C und Zugeben des physiologischen Wirkstoffs unter Mischen und
(iv) Abkühlen der Mischung aus Schritt (iii) auf eine Temperatur von 20-25 °C und gegebenenfalls Zugeben eines oder mehrerer Hilfsstoffe zu dem Gel.

## Revendications

1. Composition aqueuse de gel pour application topique comprenant
(a) un ingrédient physiologiquement actif ;
(b) une dispersion aqueuse d'éthylcellulose, et
(c) un carraghénane ;
dans laquelle la concentration d'éthylcellulose est dans la plage de 5 à 50 % en poids de la composition.

2. Composition selon la revendication 1, dans laquelle la concentration de carraghénane est dans la plage de 0,1 à 5 % en poids de la composition.

3. Composition selon l'une quelconque des revendications 1 et 2, dans laquelle le carraghénane est choisie dans le groupe constitué par l'iota-carraghénane, le kappa-carraghénane, le kappa-2-carraghénane et le lambda-carraghénane.

4. Composition selon la revendication 3, dans laquelle le carraghénane est l'iota-carraghénane.

5. Composition selon l'une quelconque des revendications 1 à 4, dans laquelle l'ingrédient physiologiquement actif est choisi dans le groupe constitué par les antibiotiques, les agents antimycosiques, les agents antiviraux, les agents anti-inflammatoires non stéroïdiens, les anesthésiques locaux, les corticostéroïdes, les rétinoïdes, les antiallergiques, les agents anticancéreux, les stimulants de la croissance des cheveux, les immunosuppresseurs, les agents antidiabétiques, les antidépresseurs, les vitamines, les agents de protection solaire, le traitement de l'alopécie, les agents anti-acné, les agents anti-psoriasiques et les agents cicatrisants.

6. Composition selon l'une quelconque des revendications 1 à 5, dans laquelle le gel a une viscosité de 100 à 6 000 mPa.s à une vitesse de cisaillement de 0,01 s⁻¹ telle que déterminée en utilisant un rhéomètre (modèle Discovery HR-3, disponible auprès de TA Instruments).

7. Composition selon l'une quelconque des revendications 1 à 6, dans laquelle le gel a une limite d'élasticité de 10 à 200 Pa déterminée comme module de stockage en utilisant un rhéomètre (modèle Discovery HR-3, disponible auprès de TA Instruments).

8. Composition selon l'une quelconque des revendications 1 à 7 qui a un pH dans la plage de 3 à **8.**

9. Composition selon l'une quelconque des revendications 1 à 8 comprenant en outre un ingrédient auxiliaire choisi dans le groupe constitué par les modificateurs de pH, les conservateurs, les tensioactifs, les émollients, les stimulateurs de pénétration, éventuellement les fragrances.

10. Composition selon l'une quelconque des revendications 1 à 9, fournissant une libération contrôlée de l'ingrédient physiologiquement actif lors de son application topique.

11. Procédé de préparation de la composition selon l'une quelconque des revendications 1 à 10, le procédé comprenant
(i) chauffer une dispersion aqueuse d'éthylcellulose jusqu'à une température de 50-80 °C,
(ii) ajouter un carraghénane à la dispersion de l'étape (i) en mélangeant continuellement jusqu'à ce qu'un gel soit formé,
(iii) refroidir le mélange de l'étape (ii) jusqu'à une température de 25-50 °C et ajouter l'ingrédient physiologiquement actif en mélangeant, et
(iv) refroidir le mélange de l'étape (iii) jusqu'à une température de 20-25 °C et éventuellement ajouter un ou plusieurs ingrédients auxiliaires au gel.
